# EUROPEAN PATENT APPLICATION

(11) **EP 2 979 622 A1**
(43) Date of publication of application: **03.02.2016**
(21) Application number: 14306223.0
(22) Date of filing: 31.07.2014
(51) Int. Cl.: A61B 3/16, G02C 7/04

(54) **Passive sensing means for a contact lens**

(71) Applicant: Ophtimalia, 14460 Colombelles (FR)
(72) Inventor: Razavet, Xavier, 14610 Cairon (FR); Moreau, Olivier, 14240 Cahagnes (FR); Pasquette, Franck, 14460 Colombelles (FR); Mezenge, Luc, 14980 Rots (FR); Cauvet, Philippe, 14000 Caen (FR); Biermans, Peter, 14112 Biéville-Beuville (FR)
(74) Representative: Grünecker Patent- und Rechtsanwälte PartG mbB

(57) **Abstract**

The invention relates to a passive sensing means (100) for a contact lens, in particular for detecting variations of a physiological parameter, comprising an inductance (101) having a plurality of concave arc-shaped segments (1011, 1012, 1013) with respect to a center point (110) of the sensing means (100), wherein the radius of curvature of at least one segment (1011, 1012, 1013) at a point thereof is greater than the distance between said point and the center point (110) of the passive sensing means (101). The invention also relates to an alternative passive sensing means with an inductive element comprising a plurality of inductances, as well as to a contact lens with incorporated passive sensing means and to a method for manufacturing said contact lens.

## Description

The present invention relates to the field of passive sensing means for contact lenses, in particular passive sensing means for measuring variations of a physiological parameter such as the intraocular pressure.

Intraocular pressure is one of the physiological parameters that allows diagnosis and monitoring of eye diseases such as glaucoma. Recently, portable and non-invasive sensing means and methods have been developed in order to measure daily variations of a patient's intraocular pressure. The continuous development of non-invasive sensing means has allowed avoiding invasive surgical procedures where sensing means would need to be implanted in a patient's eye. Furthermore, the portability of non-invasive systems has the advantage that patients are no longer required to be immobilized at a hospital or clinic, but that the physiological parameters can now be continuously monitored in daily life situations.

Non-invasive sensing means usually comprise a sensing device that can be incorporated in a contact lens to be carried by a person whose eye's physiological parameter should be monitored. Furthermore, the non-invasive sensing device can be used in combination with an external monitoring system that can receive and analyze data from the sensing means.

Different types of non-invasive sensing means for contact lenses are known in the art, among which active sensors using miniaturized low power electronics such as microchips, active strain gages and the like, and therefore requiring an energy source. WO 2011/083105 A1 discloses for instance an active sensor comprising ring-shaped concentric strain gages and an associated microprocessor that are incorporated in a ring area of a contact lens.

In contrast thereto, purely passive sensors have been developed in order to avoid using an energy source that might cause discomfort to a patient, for instance due to the generation of radiation in close vicinity of or even in direct contact with the patient's eye. A passive sensor is known for instance from EP 2 412 305 A1, which discloses a resonant LC circuit incorporated in a contact lens, in general a soft contact lens, that responds to an external magnetic field generated by a complementary portable device. In particular, EP 2 412 305 A1 discloses passive sensors comprising two superimposed and electrically connected ring-shaped circular spiral inductances. Such passive sensors rely on the fact that the resonance frequency of an incorporated LC circuit should change with the intraocular pressure, as pressure variations in the eye should affect the shape of the surface of the eye and thereby also the shape of the contact lens and, in turn, the characteristics of the incorporated passive sensor.

A portable physiological parameter monitoring system is known from EP 2 412 305 A1 and from EP 2 439 580 A1, and can comprise a spectacle frame for generating the external magnetic field and acquiring data from a contact lens incorporating a passive sensing device, as well as a base station for analyzing the acquired data.

It is generally admitted in the field of non-invasive intraocular pressure monitoring that more coverage of the surface of the patient's eye should allow providing a measurement of the average variations of the shape of the eyeball in response to variations of the intraocular pressure. However, a requirement is also that the patient's vision should not be impaired when wearing the contact lens. The sensor should also preferably have a shape that facilitates its incorporation in a contact lens.

Thus, in EP 2 412 305 A1 and in WO 2011/083105 A1, and also generally in recently developed non-invasive passive or active sensors for contact lenses of intraocular pressure monitoring systems known in the art, a ring-type geometry of the sensor has been preferred to other geometries. Finally, EP 2 412 305 A1 discloses a method for manufacturing a contact lens with an incorporated passive sensor, wherein the passive sensor is deformed to adopt the final shape of the lens.

However, during the recent development of non-invasive sensing devices for monitoring intraocular pressure variations, several problems have been encountered:
A recurrent problem, in passive and active sensors alike, is that, contrary to all expectations, the contact lens with the incorporated sensor exhibits deformed areas, such as rippled areas, in particular rippled edges, instead of having the smooth and substantially hemispherical shape expected in a contact lens. Thus, further to not being comfortable for a person wearing the lens, these deformed areas prevent a proper flat placement of the lens against the surface of the eye and, consequently, the necessary sensitivity of the system to deformations of the surface of the eyeball, and hence of the lens material, is not reached. Such deformed areas, in particular rippled edges, have also been observed in contact lenses comprising a ring-shaped passive sensor. In particular, the applicant has found out that, even if the sensor is deformed to adopt the expected shape of the lens, the resulting areas in the over-molded lens comprising the sensor are rigid, which prevents the lens from adopting its expected rounded shape and creates at least some of the deformed areas. It is believed that this problem affects most, if not all, of the known contact lenses with incorporated intraocular pressure sensors, passive and active alike. The addition of further components, for instance a microprocessor or microchip in active sensors, or the addition of capacitances in passive sensors, seems to stiffen the final lens even more.

Thus, an objective of the present invention is to provide an improved passive sensor for a contact lens of a physiological parameter monitoring system that solves the above mentioned problems, while respecting the requirements of comfort of wearing and unimpaired patient's vision. Thus, it is also an objective to provide an improved passive sensor with dimensions small enough not to impair a patient's vision, while still covering sufficient surface to allow an efficient monitoring. In particular, an objective of the present invention is also to provide a improved passive sensor that facilitates the incorporation in a contact lens and improves the placement of the contact lens against the surface of the eyeball. Thus, an objective of the present invention is also to provide an improved passive sensor with which the formation of ripples during the incorporation process in a contact lens can be at least controlled or even avoided, while providing a high sensitivity to variations of the intraocular pressure.

The objective is achieved with a passive sensing means according to claim 1, as well as with a passive sensing means according to claim 10. The objective is also achieved with a contact lens according to claim 18 and with a method for manufacturing the same according to claim 19. Optional advantageous features are described in the dependent claims, as well as in the following, and will be discussed more in detail with reference to advantageous embodiments.

According to claim 1, the passive sensing means for a contact lens, in particular for detecting variations of a physiological parameter, comprises an inductance having a plurality of concave arc-shaped segments, preferably three, with respect to a center point of the sensing means. According to this aspect of the invention, for at least one, preferably all, of the plurality of concave arc-shaped segments of the inductance, the radius of curvature of said at least one segment at a point of said segment is greater than the distance between said point and the center point of the passive sensing means.

Here, by the expression "arc-shaped" or the like, it should be understood that each arc-shaped inductance segment has, respectively, a curved geometry that follows essentially the shape of an arc of a circle or, more generally, of an arc of an ellipse. Furthermore, while each arc-shaped segment can preferably be a continuous arc-shaped segment, a plurality of shorter back-to-back linear segments could also realize one longer segment having a globally arc-shaped geometry, which would also allow carrying out the invention. Also, by the expression "concave with respect to a center point" and the like, it should be understood that the arc-shaped segments are all concave facing a same center point of the sensing means. According to the invention, this point is not the center of the arc-shaped segments but merely indicates a respective direction with respect to which the arc-shape segments are concave. Thus, according to the invention, the concave arc-shaped segments are not on a circle centered on the center point. It has been found out that an inductance with such concave arc-shaped segments facing a geometrical center point of the passive sensing means, having a respective curvature radius larger than the distance to said geometrical center, is easier to incorporate in a contact lens than sensors comprising ring-shaped inductances known in the art.

Indeed, the inventive passive sensing means has an inductance with a structure comprising a plurality, preferably three, flap or ear-like segments that can be better adapted to the spherical cap shape of a contact lens than the circular ring-like inductances of sensors known in the art because they allow controlling the areas of the passive sensing means that will be bent, folded and/or plastically deformed during the incorporation and therefore also controlling and even avoiding the formation of ripples. In particular, the concave segments of the inductance provide a solution to the problem of adapting a sensor manufactured with a flat 2D-structure (the sensor before incorporation in a contact lens) to the 3D-structure of a contact lens (a spherical cap). Given the dimensions of contact lenses and therefore the requirements on the dimension of passive sensing means, three concave arc-shaped inductance segments provide a better compromise in terms of sensitivity and surface covered by the inductive element of the passive sensing means, as well as in terms of flexibility for the incorporation of the sensor in the contact lens than more or less such segments. However, two, four or more concave arc-shaped segments with large radii also allow carrying out the invention and should therefore not be ruled out.

Furthermore, it is possible and even preferred that the curvature radii of the concave arc-shaped segments of the inductance are chosen such that, once the passive sensing means is deformed for its incorporation in a contact lens, they will essentially describe segments of a same predetermined circle of the contact lens, essentially parallel to the small circle of the lens. In this manner, ripples are essentially avoided in the final contact lens, which presents an important advantage in comparison to prior art sensors.

Preferably, the inductance can further comprise convex arc-shaped segments arranged between the concave arc-shaped segments. Here, the expression "convex arc-shaped segments" should be understood in a manner similar to "concave" as explained above. Thus, the convex arc-shaped segments are convex with respect to a center point of the sensor, as explained above, which is also a reference for the concave arc-shaped segments. In this way, the areas where the passive sensing means is bent during the incorporation process, which are the areas that could produce ripples in the final contact lens, are controlled. The depth of the inwards extending ear-like structures formed by arc-shaped inductance segments that are convex with respect to the center point can be chosen so as to reduce and even avoid the formation of ripples in the contact lens with incorporated passive sensing means. Also these convex segments form areas the size of which can be chosen to leave sufficient space to bend the sensor during the incorporation process without creating ripples.

In a variant of an advantageous embodiment, the inductance can further comprise straight segments joining the convex arc-shaped segments to the concave arc-shaped segments. The length of the joining straight inductance segments can be used to better control the amount of material between the concave arc-shaped segments and thereby also improve the control over the formation of ripples in the over-molded contact lens.

Advantageously, the junctions between the straight segments and the concave arc-shaped segments are rounded. This variant is advantageous, as rounded junctions between successive inductance segments provide smoother shapes than rough pointy edges. Here, attention should be brought to the fact that, while in this variant the rounded junctions could thus be concave-shaped, they are however not concave "with respect to the center point", unlike the "concave arc-shaped segments" as explained above.

Preferably, the inductance can be a spiral inductance. Thus, a flat structure can be manufactured by depositing a conductive material in or on a carrier substrate following a spiral. Advantageously, the inductance can comprise 5 to 20 spires, preferably 8 to 15 spires, more preferably 10 to 13 spires. Also, in preferred embodiments of this variant, the width of the spires and/or the distance between spires can be in a range from about 30 µm to about 100 µm, preferably about 40 µm to about 80 µm. Thus, the invention allows combinations of number of spires and dimensions that can advantageously allow a patient to keep a clear vision while wearing a contact lens with the inventive passive sensing means. In particular, it is possible but not necessary that the width of the spires and the distance between successive spires are the same.

Advantageously, the width of the inductance is about 2 mm or less, preferably about 1.5 mm or less. The width of the inductance can in fact be greater than this value, but it is more advantageous that it is kept lower in order to keep the patient's vision clear, as well as to keep the amount of material in the sensor to a reasonable amount that can be deformed and incorporated in a contact lens without creating ripples or stiffened structures that would deteriorate the sensitivity of the sensor and incommode or even hurt a patient wearing a contact lens with an incorporated sensor. Thus, a preferred and advantageous variant can adapt the number and size of the spires in order to match this requirement for the width of the inductance.

Furthermore, the inductance can be provided in or on a carrier substrate, and the carrier substrate can be removed following a predetermined pattern, preferably following an external contour and/or an internal contour of the passive sensing means. The problem of incorporating the passive sensing means in a contact lens is somewhat similar to wrapping a 3D surface with a 2D sheet. It is therefore advantageous to remove areas of carrier substrate that would create unnecessary material and therefore form ripples when deforming the passive sensing means to give it a curved shaped prior to its incorporation in a contact lens. By avoiding ripples on the deformed carrier substrate, it is also possible to better avoid consequent ripples in the over-molded contact lens. Thus, the unnecessary carrier substrate can be removed, for instance using a laser, following a predetermined pattern around the passive sensing means, preferably following the external contour thereof and/or also following the internal contour thereof, in other words the carrier substrate can also be removed in the central area of the inductance. Thus, it is possible to remove as much carrier substrate as possible in order to make the passive sensing means as flexible as possible prior to its incorporation in a contact lens.

In an advantageous variant, more substrate can be left in areas of the internal contour corresponding to the junction between the convex arc-shaped segments and the concave arc-shaped segments. Since these areas form acute angles and are therefore subject to possible tears, it is advantageous that, instead of strictly following the internal contour of the inductance when removing the carrier substrate, small detours are made in order to leave more substrate in these areas.

Alternatively, according to claim 10, the passive sensing means for a contact lens, in particular for detecting variations of a physiological parameter, comprises an inductive element with a plurality of inductances. According to the invention, the inductances are spaced apart from one another, preferably in a non-overlapping manner, in particular in a projection onto the small circle of the contact lens.

It has been found out that a passive sensor with "satellite" inductances that are spaced apart from each other, preferably in a non-overlapping manner is also easier to incorporate in a contact lens than the sensors known in the art, in particular than the ring-shaped sensors known in the art. Indeed, by having inductances that, in the resulting contact lens, will be distributed in the lens, preferably evenly and not overlapping with one another, the over-molding of the lens is facilitated compared to the prior art. In the following, the expression "satellite inductances" can also be used to designate the individual inductances of the inductive element, which are distributed in a non-overlapping manner, in contrast to piled-up inductances of sensors known in the art. In particular, it is possible to choose the distribution and shape of these satellite inductances such that, in the resulting lens, they are used to facilitate the positioning of the lens flat against a person's eyeball. Thus, the inductive element of this aspect of the invention plays a similar role to the inductance of the passive sensing means according to claim 1. Furthermore, in terms of the incorporation or over-molding process of the sensor in a contact lens, the satellite inductances play an analogous role to the ear or flap-like concave arc-shaped inductance segments of the previous aspect of the invention, as they both facilitate the process in a similar way.

A contact lens can be roughly described as substrate shaped as a spherical cap, wherein the thickness of the substrate can be the same over the entire spherical cap or wherein the edges can be thinner relatively to a thicker center part, the latter being more common and more advantageous for a person's comfort and vision. A contact lens comprises an internal optical surface, also referred to as "internal surface" hereafter, which is the surface destined to be in contact with a person's eyeball, and an external optical surface, also referred to as "external surface" hereafter, which is the surface facing outwards from the person's eyeball and thus not in direct contact with it but that may contact the inside of the eyelid.

Furthermore, a "circle of a sphere" is known to be a circle defined by the intersection of said sphere with a plane. When the intersecting plane contains the center of the sphere, the circle is called a "great circle" or a "meridian". Otherwise, it is called a "small circle".

Since a contact lens can typically be assimilated to a spherical cap similar but smaller than a full hemisphere, in this application expressions such as "small circle of the contact lens" and the like shall be used in order to refer to the circle defined by the edges of the contact lens. Thus, the plane onto which the projection is done is also the plane defining the small circle of the lens.

Furthermore, the points where the external and the internal optical surfaces, respectively, of a contact lens cross the optical axis of the lens will be referred to as the "external surface vertex" or "external vertex" and as the "internal surface vertex" or "internal vertex", respectively, in the following. The projections of the external and of the internal vertices onto the plane of the small circle fall on the center of the small circle.

In passive sensing devices known in the art, the center of the circular ring-shaped spiral inductance(s) is aligned with the external and internal vertices on the optical axis of the contact lens. When more than one inductances are used in the prior art, they are piled up like in EP 2 412 305 A1, and then the centers of both inductances fall on the optical axis of the lens. However, as explained above, this ring-like geometry creates an unexpected stiffness of the over-molded contact lens and causes at least some of the deformations of the lens, including at least some of the observed ripples that prevent the proper flat placement of the lens. In contrast, the present invention has the advantage over the known prior art that the flexibility of the lens is improved by having one or more inductances spaced apart from one another, such that they do not form a stiff ring area in the contact lens. Thus, the inventive sensing device advantageously allows, after the over-molding of a contact lens, a geometry that is more favorable to the proper flat placement of the contact lens against a patient's eyeball.

In a preferred embodiment, a plurality of spaced apart and non-overlapping essentially flat spiral inductances have been found to be advantageous in this respect in comparison to known non-invasive sensors for contact lenses used in physiological parameter monitoring systems. In particular, instead of using a ring-shaped sensor comprising ring-shaped spiral inductances, this aspect of the invention allows using a plurality of satellite spiral inductances, preferably circular, spaced apart from one another. Depending on the size of the inductances, distributions of two, three, four or even more inductances could be used in order to increase the sensitivity of the passive sensing means and cover more of the surface of the lens and, consequently, of the underlying surface of the eye. Like in the previous aspect, three or four inductances have been found advantageous regarding surface coverage, and hence global sensitivity, versus flexibility during the over-molding process.

A passive sensing means according to any aspect of the invention could also comprise one or more capacitors. However, in contrast to passive sensing means known in the art, additional "physical" capacitors are merely optional, as the inductance(s) allows the inventive passive sensing means to rely on variations of the value of the intrinsic "parasitic" capacitance of the inductance(s) as sole capacitive parameter in the resonant LC circuit. Thus, it is still possible, but not preferred, to use capacitors in a passive sensor, provided that the inductance(s) are chosen such that the resonance frequency of the passive sensing means is in an accepted range for medical uses. Furthermore, by avoiding the necessity of using physical capacitors to realize the resonant LC circuit, the inventive passive sensing means intrinsically also avoids any problem due to a possible misalignment of the capacitor's electrodes and additional components that would introduce stiff areas in the contact lens.

Preferably, the inductances can be off-centered with respect to the center of the sensing means. In particular, in the projection onto the small circle of the contact lens, the satellite inductances can be off-centered with respect to the center of the small circle. Thus, the vision of a patient wearing a contact lens with the incorporated inventive passive sensing means will not be impaired.

In a preferred embodiment, the inductance(s) can be arranged on the same side of a carrier substrate. Thus, the relative positioning of the inductance(s) can be defined prior to the incorporation of the passive sensing means in a contact lens, in particular, when the passive sensing means is still flat. In one aspect, the satellite inductances can then be coplanar and spaced apart from each other, preferably in a non-overlapping manner so to avoid creating any unnecessarily stiffened area.

Advantageously, the center of each satellite inductance in the plurality can be equidistant from the center of the sensing means, in particular from the center of the contact lens in the projection. In other words, when the passive sensing means is flat, prior to being curved for its integration in a contact lens, or when the integrated passive sensing means is projected onto the plane of the small circle, the inductances can be equidistant from the center of the small circle. In yet other words, the respective center of each inductance can be equidistant from the optical axis. Embodiments with a passive sensing means geometry in which several inductances are all spaced apart from one another and equidistant from the optical axis has been found advantageous, as it allows for strategic placement of each inductance in the lens, thereby improving the surface covered by the sensing device with respect to sensors known in the art.

Further to being off-centered, the inductances can also preferably not overlap with a central area of the sensing means or of the optical surfaces of the subsequently over-molded lens or with a projected central area in the projection onto the small circle. In a preferred embodiment, the central area can be a predetermined central area. In particular, the central area can be essentially circular. Thus, it is possible to avoid the central part of a contact lens that will correspond to the pupil of a person wearing the lens and keep the patient's vision essentially clear.

Advantageously, the central area can be of a predetermined size, preferably corresponding at least to about a person's average smallest pupil size, so that the vision of a patient can remain essentially clear. In a preferred embodiment, the central area can have, for instance in the projection onto the small circle, dimensions corresponding to at least a small human pupil diameter under bright daylight conditions, for instance at least about 2 to 3 mm.

Advantageously, the inductances can be connected to each other with the connections being arranged preferably towards the center of the sensing means. In this case, the connections can preferably be bridge-like connections. In a preferred embodiment, when the inductances are on the same side of a carrier substrate, the connections can be either on the other side of the carrier substrate or on another carrier substrate. In a further preferred embodiment, it can be advantageous to arrange the connections towards the central area of the passive sensing means. An embodiment in which the inductances would work as a plurality of individual non-electrically connected passive sensing means, covering each a different part of the eye and responding to one or more external magnetic fields is also possible. However, it is more advantageous when the inductances are electrically connected to each other, thereby forming a single and more sensitive passive sensing means, responding to a single external magnetic field. In an even more advantageous variant, all the inductances can be similar in shape, size and, in a variant in which the inductances are spiral inductances, the spiraling direction can be the same.

In a preferred embodiment of an aspect of the invention, the satellite inductances can be connected in parallel such that, when the inductances are placed in the magnetic field of an associated external monitoring system, the outgoing currents generated by the inductances exit through a same first node, while the ingoing currents enter through a same second node, different from the first node. This configuration has been found to increase, in particular maximize, the sensitivity of the inventive passive sensing device with respect to sensors known in the art. The parallel connections can be achieved using bridge-like elements connecting each inductance to a neighboring inductance. In particular, said bridge elements can be arc-shaped such that a central area corresponding to person's pupil will not be covered and the patient's vision will not be hindered.

In a preferred variant of the previous embodiment, at least one of the connections can be left opened. When the inductances are connected in parallel, for instance with bridge-type connections arranged towards a central area of the passive sensing means, it has been observed that the "open loop" created by leaving at least one of the parallel connections between two neighboring inductances open can advantageously prevent the formation of Foucault currents, also known as eddy currents, which would perturb the monitoring.

In yet another preferred embodiment, the inductances can be spaced apart at regular intervals, in particular at intervals of 360 / N degrees, wherein N is the total number of inductances. Since the passive sensing means will be incorporated in an essentially spherical cap that is the contact lens, in order to facilitate the incorporation and avoid any asymmetry in the resulting lens, an even distribution of the plurality of inductances has been found advantageous compared to inductances arranged at irregular intervals. Furthermore, it has been observed that a regular spacing between the inductances also increases the sensitivity of the inventive passive sensing device, as the coverage of a patient's eyeball surface can be improved with respect to that of sensors known in the art.

Preferably, the inductances can be spiral inductances, in particular circular spiral inductances, more in particular having substantially the same size. Embodiments with classic, non-ring-shaped, circular spiral inductances have been found advantageous compared to other geometries, and in particular compared to inductances used in passive sensors known in the art. The invention, however, also works with other geometries than circular-shaped spiral inductances, for instance square-shaped or star-shaped spiral inductances and the like. It is also advantageous regarding the overall sensitivity of the passive sensing means when the inductances, further to having the same shape, also have the same dimensions.

In a preferred embodiment of this aspect of the invention, when the inductances are provided on a carrier substrate, the carrier substrate can be removed at least in areas between the inductances, preferably following a predetermined pattern, more preferably following the contour of the passive sensing means. The problem of incorporating the passive sensing means in a contact lens is somewhat similar to wrapping a tri-dimensional surface with a two-dimensional sheet or to manufacturing a hot-air balloon from a flat piece of fabric. It is therefore advantageous to remove areas of carrier substrate that would create ripples when deforming the passive sensing means to give it a curved shaped prior to its incorporation in a contact lens. By avoiding ripples on the deformed carrier substrate, consequent ripples in the over-molded contact lens can also be avoided. Like in hot-air balloons or wrapping methods, the unnecessary carrier substrate can be removed following a petal-shaped pattern, wherein the petals can follow curved segments, preferably corresponding to projected great circular arcs in the lens, or straight radial segments. Optionally, it is also possible to remove the carrier substrate following more complex patterns, including removing additional areas of substrate along the curved or straight petal segments. It is also possible to remove the carrier substrate following the contours of the passive sensing means, and thereby remove preferably as much carrier substrate as possible in order to make the passive sensing means as flexible as possible prior to its incorporation in a contact lens.

The object of the invention is also achieved by a contact lens according to claim 18, comprising a passive sensing means according to any of the previous aspects, and by a method according to claim 19 for manufacturing said contact lens. Further advantageous features and method steps are described in the dependent claims and are optional. The method comprises the steps of: providing a passive sensing means according to any of the previous aspects; and over-molding a contact lens over the passive sensing means, such that the passive sensing means is incorporated in the contact lens between an internal optical surface and an external optical surface of the contact lens, wherein the internal optical surface is the surface for contacting a person's eyeball, and the external optical surface is the outer surface opposite the internal surface.

The various embodiments of the inventive passive sensing means and their variants have the advantage over sensors known in the art that the over-molding of the lens will not result in stiffened areas or ripples that will cause problems for placing the lens against a person's eye. Instead of the ring-shaped stiff area and the ripples on the edge observed in known sensors and contact lenses, advantageous embodiments of the contact lens manufactured in particular by the inventive method will comprise a series of concave arc-shaped inductance segments or satellite inductances arranged around a central area, creating regions that will help pressing the lens against the surface of the eye. According to the present invention, these elements will form ear or flap-like elements that will facilitate the over-molding of the lens. Thus, following the inventive method, it is possible to manufacture a contact lens comprising the inventive passive sensing means and therefore presenting essentially the same advantages.

In a variant, the method can further comprise, preferably prior to the step of over-molding the contact lens, a step of deforming, in particular plastically, the passive sensing means to obtain a curved shape corresponding to the shape of the contact lens. It is imaginable to over-mold a contact lens over the inventive passive sensing means and then force the lens to adopt a spherical shape, but ripples could be formed. It is thus preferably to deform the passive sensing means prior to its incorporation in the lens. In combination with advantageous variants of the inventive passive sensing means, the formation of ripples in the contact lens can be avoided.

For instance, in combination with an inventive passive sensing means provided in or on a carrier substrate, the method can further comprise, prior to the step of over-molding the contact lens or to the step of deforming the passive sensing means, a step of removing the carrier substrate following a predetermined pattern. Thus, according to one aspect, the carrier substrate can be removed at least in areas between the satellite inductances, preferably following a predetermined pattern, more preferably following the contour of the passive sensing means. Similarly, according to another aspect, the carrier substrate can be removed following at least the external contour of the inductance, and optionally also in the central area thereof, following the internal contour of the inductance. As explained above in relation to a corresponding variant of the inventive passive sensing means, removing the carrier substrate following predetermined patterns has the advantage that the formation of ripples can be avoided in the passive sensing means and, consequently, in the over-molded contact lens. Preferred predetermined patterns can be followed to remove the carrier substrate, such as petal-shaped patterns. An even more preferred pattern is following the contours of the passive sensing means, as long as sufficient substrate is left in areas that could otherwise risk being torn during the incorporation process.

Thus, in preferred combinations of advantageous variants of aspects of the invention, it is possible to choose the distribution of the arc-shaped inductance segments or of the satellite inductances in the passive sensing means, in particular in order to allow removing the carrier substrate following advantageous patterns, thereby improving the incorporation of the passive sensing means in the contact lens in comparison to known contact lenses with incorporated sensors. In particular, the invention improves the placement of the contact lens against the surface of the eyeball, which in turn improves the response of the incorporated passive sensing means to deformations of the surface of the eye due to variations of the intraocular pressure, and, consequently, the efficiency of the physiological parameter monitoring system.

The invention will be described in more detail in the following, based on the above and on advantageous embodiments described in combination with the following figures, wherein:
- Figure 1: schematically illustrates a passive sensing means in an exemplary embodiment of the first aspect of the invention;
- Figure 2: schematically illustrates the passive sensing means of the exemplary embodiment illustrated in Figure 1, wherein a carrier substrate is removed around the passive sensing means;
- Figure 3: schematically illustrates a passive sensing means in a first exemplary embodiment of the second aspect of the invention;
- Figure 4: schematically illustrates an example of connections between the inductances of the passive sensing means illustrated in Figure 3;
- Figure 5: schematically illustrates a second exemplary embodiment of the second aspect of the invention, wherein a carrier substrate is removed around the passive sensing means following a predetermined pattern; and
- Figure 6: schematically illustrates a third exemplary embodiment of the second aspect of the invention, wherein a carrier substrate is removed following the contour of the passive sensing means.

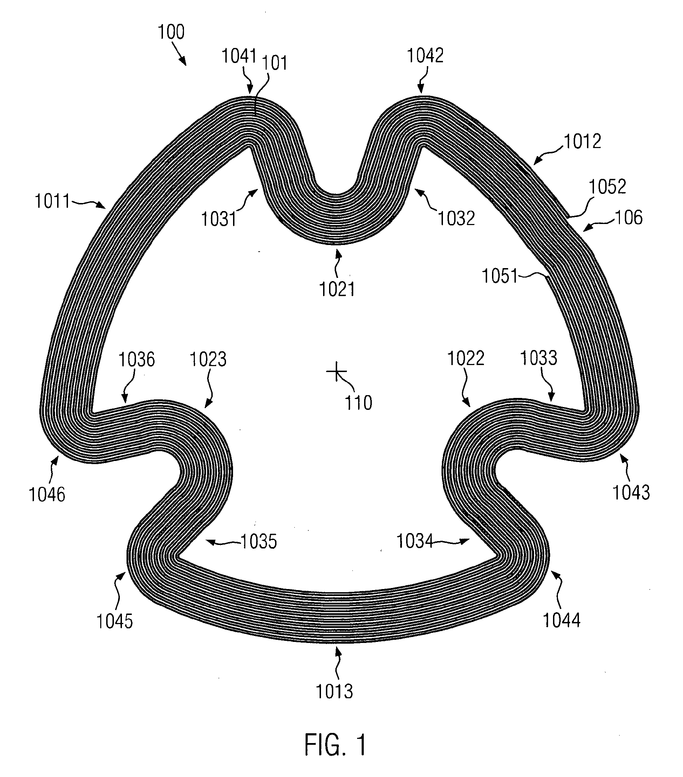

Figure 1 illustrates a passive sensing means 100 for a contact lens used in a physiological parameter monitoring system, in an embodiment of the first aspect of the invention. In this embodiment, the passive sensing means 100 is a resonant sensor that will be incorporated in a contact lens for detecting variations of a physiological parameter in a person's eye when the contact lens is worn by said patient. In particular, the passive sensing means 100 illustrated in Figure 1 can be used in a contact lens for monitoring variations of the intraocular pressure in a person's eye suffering from glaucoma.

As can be seen in Figure 1, the passive sensing means 100 comprises an inductance 101 which, according to this aspect of the invention, comprises a plurality of segments 1011, 1012, 1013 that are all arc-shaped and concave with respect to a same reference center point 110 of the inductance 101 or of the sensor 100. However, as further illustrated in Figure 1, these segments 1011, 1012, 1013 are in fact not centered on said center point 110. Indeed, at least one segment 1011, 1012, 1013, and preferably all three segments 1011, 1012, 1013, has a curvature radius at a point thereof that is greater than the distance of said point to the center point 110. Thus, following a preferred variant, the centers of the concave arc-shaped inductance segments 1011, 1012, 1013 can in fact be even outside of the inductance 101. By not being circular, the inductance 101 has the advantage that the ear-like structure of the three segments 1011, 1012, 1013 will be easier to incorporate in the spherical cap shape of a contact lens. In fact, it will be even possible to bend the sensor 100 such that the segments 1011, 1012, 1013 will fall onto a predetermined (small) circle of the contact lens, parallel to the small circle as defined above.

As also illustrated in Figure 1, in order to further facilitate the incorporation process, in particular to better control the areas that will bend during this process, the inductance 101 of the passive sensing means 100 can further comprise inwards orientated, in other words convex, arc-shaped segments 1021, 1022, 1023 joining the concave segments 1011, 1012, 1013. Depending on the desired size of the passive sensing means 100, Figure 1 also illustrates that it is possible to join the concave segments 1011, 1012, 1013 to the convex segments 1021, 1022, 1023 via straight inductance segments 1031, 1032, 1033, 1034, 1035, 1036. Thus, the depth of the inwards pointing ear-like segments 1021, 1022, 1023 can be adjusted, thereby controlling the areas that will be bent during the incorporation process, which, in turn, allows better control and even prevention of any ripples in the contact lens. Figure 1 also illustrates an advantageous variant in which the junctions 1041, 1042, 1043, 1044, 1045, 1046 between the straight segments 1031, 1032, 1033, 1034, 1035, 1036 and the concave segments 1011, 1012, 1013 are rounded in order to provide a smoother shape for the incorporation process.

Following a preferred variant, the inductance 101 can be a spiral, in particular flat, inductance. Figure 1 illustrates that the inductance 101 can thus spiral from a first terminal 1051 on the inner circumference of concave arc-shaped segment 1012 towards a second terminal 1052 on the outer circumference thereof. In order to keep segment 1012 globally arc-shaped, the area 106 between the two terminals 1051, 1052 can present a small deflection, as illustrated in Figure 1.

Following yet another variant, the inductance 101 can comprise 5 to 20 spires, preferably 8 to 15 spires, more preferably 10 to 13 spires. In the embodiment illustrated by Figure 1, the inductance 101 comprises for instance 10 spires. Furthermore, since it is preferably that the width of the inductance 101 is kept below about 2.0 mm, or even at about 1.5 mm or below, in the embodiment illustrated by Figure 1, the width of the spires could be about 60 µm, while the distance between spires could be about 75 µm. However, in other embodiments, the width of the spires and/or the distance between spires could be chosen in the range from about 30 µm to about 100 µm, preferably between about 40 µm and about 80 µm. In some embodiments, they could be the same. For instance, it would be possible to have 15 spires with a width of about 50 µm and with a distance therebetween of also about 50 µm.

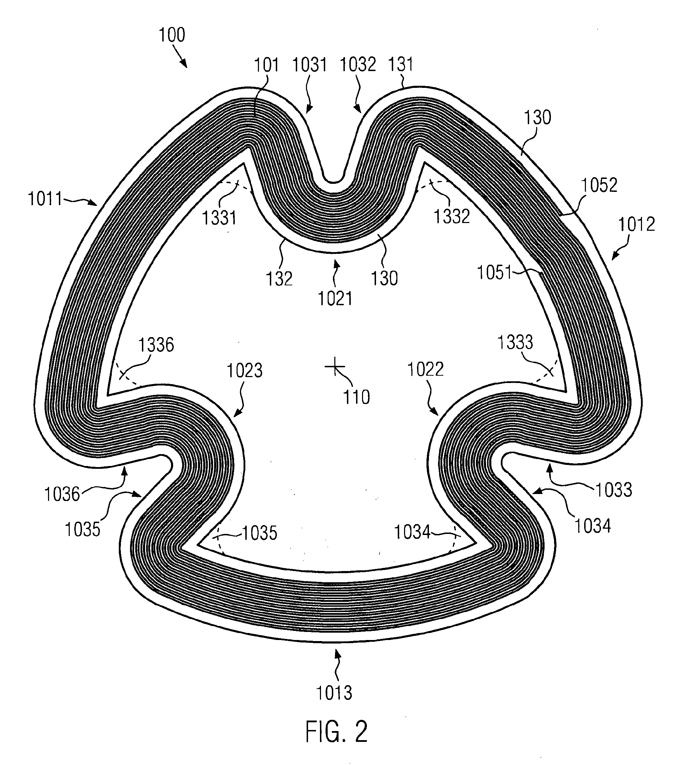

Furthermore, the inductance 101 can be a conductive material deposited in or on a carrier substrate 130. In order to remove unnecessary material that could create ripples in the bent structure of the passive sensing means 100 during the incorporation process, the carrier substrate 130 can be removed following a predetermined pattern. Figure 2 illustrates the passive sensing means 100 of the embodiment illustrated in Figure 1, with a carrier substrate 130 removed following the external contour 131 of the inductance 101. Alternatively, or in complement thereto, the carrier substrate can also be removed following the internal contour 132 of the inductance 101, as also illustrated in Figure 1.

Figure 2 also shows areas 1331, 1332, 1333, 1334, 1335, 1336 in the acute angled spaces formed by the junction between the straight inductance segments 1031, 1032, 1033, 1034, 1035, 1036 and the concave arc-shaped inductance segments 1011, 1012, 1013. Given the acute angle thereof, these areas 1331, 1332, 1333, 1334, 1335, 1336 can be torn during the incorporation process, when the passive sensing means 100 is bent to adopt the spherical cap shape of a contact lens. Thus, it is preferable not to remove the substrate 130 from these areas 1331, 1332, 1333, 1334, 1335, 1336 in order to avoid tears in the substrate 130 and/or in the junction areas 1041, 1042, 1043, 1044, 1045, 1046 of the inductance 101.

Finally, in the embodiment illustrated in Figures 1 and 2, when the passive sensing means 100 is incorporated in a contact lens, the concave arc-shaped segments 1011, 1012, 1013 can be flapped outwardly, while the convex arc-shaped segments 1021, 1022, 1023 can be flapped inwardly such that they follow the spherical cap shape of the contact lens.

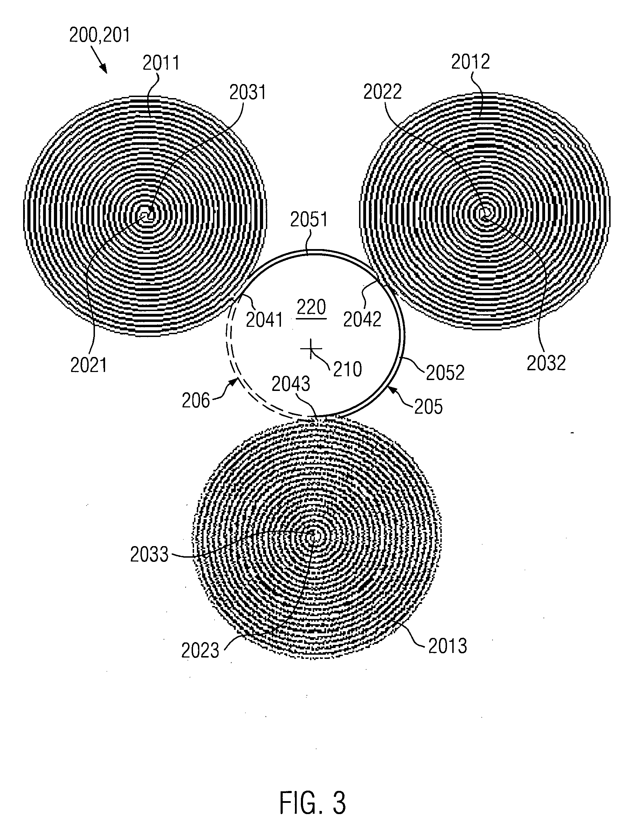

Figure 3 illustrates a passive sensing means 200 for a contact lens of a physiological parameter monitoring system, in a first embodiment of the second aspect of the invention. In this embodiment, the passive sensing means 200 is a resonant passive sensing means that will be incorporated in a contact lens for detecting variations of a physiological parameter in a person's eye when the contact lens is worn by the patient. In particular, the passive sensing means 200 illustrated in Figure 3 can be used in a contact lens for monitoring variations of the intraocular pressure in a person's eye suffering from glaucoma.

As can be seen in Figure 3, in the first embodiment of the second aspect of the invention, the passive sensing means 200 comprises an inductive element 201 having a plurality of satellite inductances, here the three inductances 2011, 2012, 2013 that are spaced apart from one another. The number of inductances can vary in other embodiments, as long as the inductances have sufficient space to be spaced apart from each other, in particular when the sensor is incorporated in a contact lens. In particular, the inductances 2011, 2012, 2013 are spaced apart along the circumference of the inductive element 201 of the passive sensing means 200, but they are not piled up or superimposed like in prior art passive sensors. Thus, the arrangement of the satellite inductances 2011, 2012, 2013 can be compared to a petal-like arrangement. According to a preferred variant of this aspect of the invention, the inductances 2011, 2012, 2013 can be arranged such that an area 220 can be left free around a center point 210 of the sensing means 200. Figure 3 illustrates the passive sensing means 200 as it would be seen if provided in or on a first substrate, prior to any deforming and/or over-molding step for incorporating the sensing means 200 in a contact lens. Thus, in Figure 3 the passive sensing means 200 can be seen in a plane similar to a projection plane onto the small circle of a contact lens. Thus, in Figure 3, the center 210 of the passive sensing means 200 is, in this case, also the center of the small circle in the projection. When the passive sensing means 200 is deformed, in particularly plastically, thereby adopting a curved shape, and subsequently incorporated in a contact lens, its center 210 is on the optical axis of the lens and is therefore aligned with the external and internal surface vertices of the lens. The central area 220 around the center 210 can then provide for a clear and essentially unimpaired vision of a subject wearing the contact lens.

According to a further preferred variant of this aspect of the invention, in the projection plane, which can for practical reasons be assimilated here to the plane defined by Figure 3, the centers 2021, 2022, 2023 of the inductances 2011, 2012, 2013 can be equidistant from one another, in particular they can be equidistant from the center 210. Furthermore, following yet another preferred variant, the centers 2021, 2022, 2023 can be off-centered with respect to the center 210 in the projection, such that the size of the central area 220 which is left free will be enough to allow proper vision of a patient wearing a contact lens equipped with the passive sensing means 200. Furthermore, following yet another preferred variant, Figure 3 also illustrates that the satellite inductances 2011, 2012, 2013 can be arranged such that they not overlap with each other in the projection plane. In a preferred embodiment, the central area 220 can correspond to a patient's average pupil size, such that the preferred configuration illustrated in Figure 3 will not hinder the patient's vision. In particular, the central area 220 can be such that at least an area of about the smallest diameter of a human pupil under bright daylight conditions is left free, for instance an area of at least about 2 to 3 mm in diameter.

As illustrated in Figure 3, the satellite inductances 2011, 2012, 2013 of the inductive element 201 can preferably be spiral inductances, in particular spiraling from a respective inner pole 2031, 2032, 2033, being a point having substantially the smallest radius of the spiral inductance 2011, 2012, 2013, towards a respective outer pole 2041, 2042, 2043, being a point having substantially the largest radius of the spiral inductance 2011, 2012, 2013. Figure 3 illustrates that the inner poles 2031, 2032, 2033 are arranged, respectively, in vicinity of the center 2021, 2022, 2023 of the satellite inductance 2011, 2012, 2013. However, in other embodiments, the inner poles 2031, 2032, 2033 could also be arranged on a respective center 2021, 2022, 2023.

In the plane of Figure 3, which corresponds also to the projection plane or to the plane on which the inductances 2011, 2012, 2013 are provided in or on a carrier substrate (not illustrated for clarity purposes), the spiral orientation can advantageously be the same for all three inductances 2011, 2012, 2013, thereby increasing the sensitivity of the passive sensing means 200. Figure 3 also illustrates that the inductances 2011, 2012, 2013 are circular-shaped. However, the skilled person will understand that other shapes are possible such as rectangular or star-shaped spiral inductances or the like.

In other embodiments, it would be possible to have different sizes and shapes for each inductance of the plurality. It is, however, advantageous that, like in the embodiment illustrated in Figure 3, all the satellite inductances of the inductive element of a passive sensing means according to the second aspect of the invention have essentially the same size and shape. For instance, the resonance frequency of all the inductances will be essentially the same, whereas it could be different if the size and/or shape of the inductances were different.

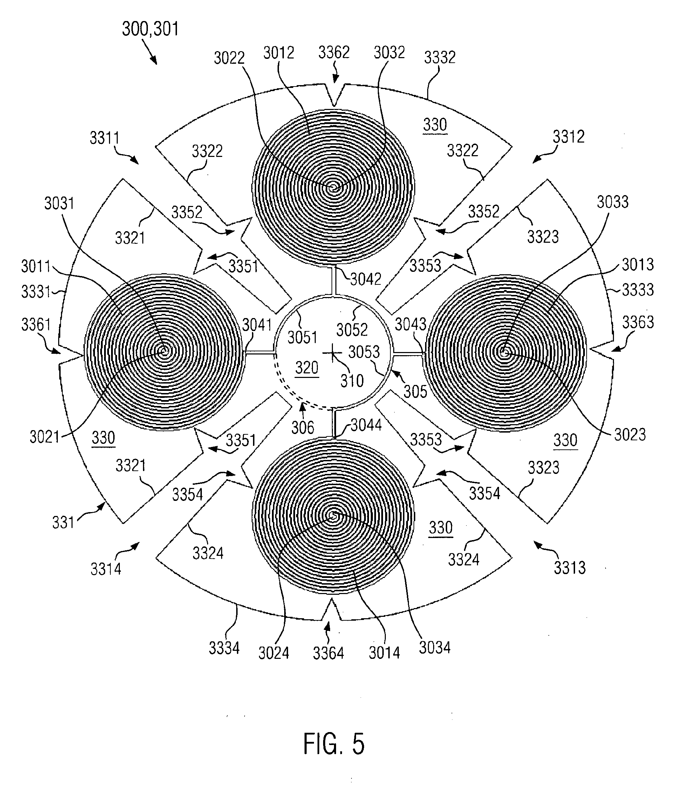

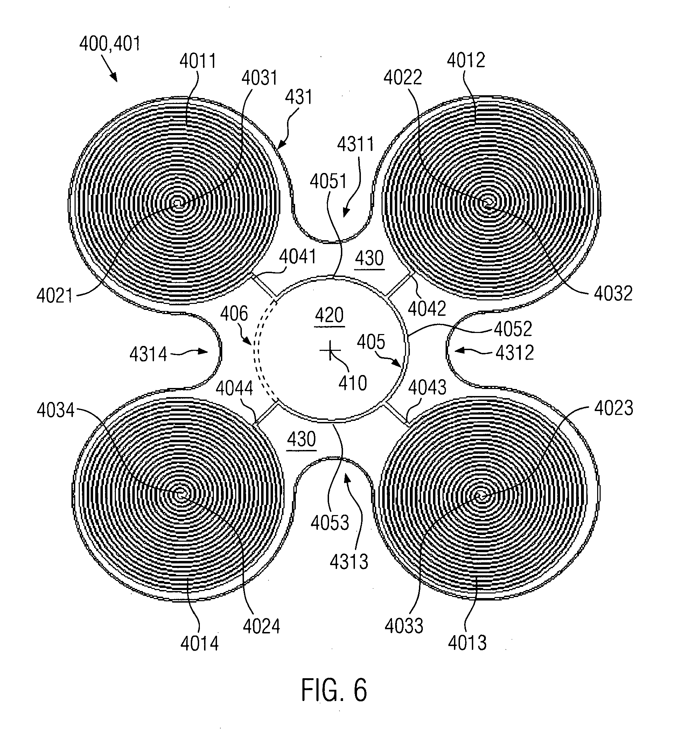

In general, in variants of the inventive sensing means, it is also advantageous that the satellite inductances are spaced apart in a regular manner, as this allows a regular coverage of the surface of the eye when the sensing means is incorporated in a contact lens that is being worn by a subject for monitoring purposes. For instance, when N is the total number of inductances in the plurality of inductances comprises in a passive sensing means according to the invention, the inductances can be spaced apart from one another by 360 / N degrees. This distribution is particularly advantageous in combination with inductances that have the same size and shape in order to allow sufficient coverage of the surface of a patient's eyeball, but inductances of different sizes and shapes could be also used in less regular distributions. In the embodiment illustrated in Figure 3, the passive sensing means 200 comprises the three inductances 2011, 2012, 2013, which can preferably be spaced apart in a regular manner, here by 360 / 3 degrees = 120°, from one another. In an embodiment with four inductances, for example like in the embodiments illustrated in Figures 5 and 6, the satellite inductances can then preferably be spaced apart by 360 / 4 degrees = 90°. Since the invention allows also using inductances of different sizes and shapes, the angular or spatial distribution could be different than this regular distribution. However, a distribution providing a regular coverage of the eyeball surface underlying the contact lens is advantageous for the purposes of monitoring variations of a physiological parameter such as the intraocular pressure, as well as for allowing a better placement of the final over-molded contact lens with incorporated passive sensing means flat against the surface of a subject's eye.

As can be seen in Figure 3, following yet another advantageous variant, the inductances 2011, 2012, 2013 of the passive sensing means 200 can be connected to each other. A connection in series or in parallel can be used. In the first embodiment, a parallel connection 205, 206 is chosen following a preferred variant, as it advantageously improves the sensitivity of the passive sensing means 200 with respect to sensors known in the art. The parallel connection 205, 206 further has the advantage that, when the passive sensing means 200 is in the magnetic field of an external monitoring device, the outgoing currents generated by the plurality of inductances 2011, 2012, 2013 exit through a same first node of the inductive element 201, while the ingoing currents enter through a same second node, different from the first node, which improves, and even maximizes, the sensitivity of the inventive passive sensing means 200 in comparison to sensors known in the art.

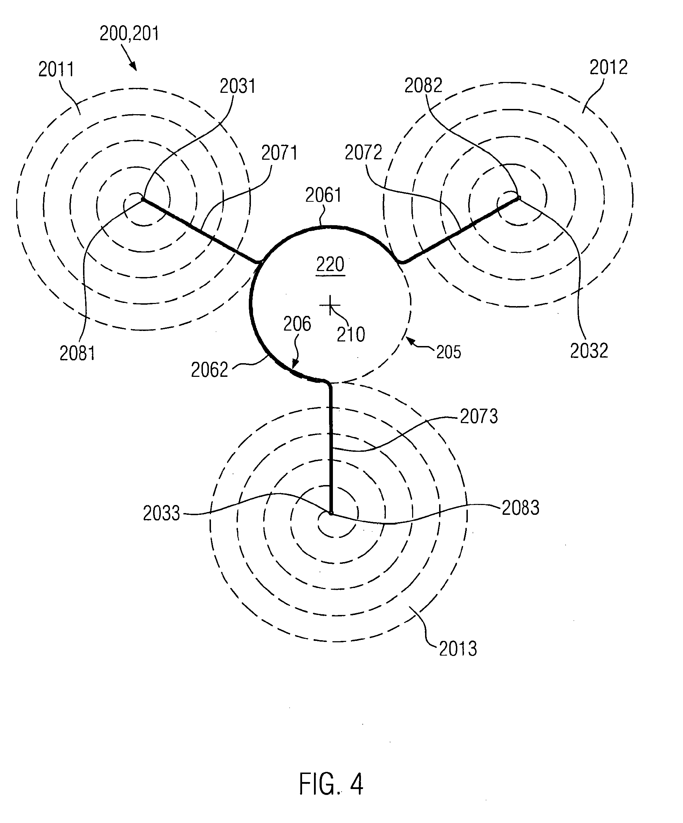

The connection 205, 206 between the inductances 2011, 2012, 2013 will be explained also with reference to Figure 4, showing the passive sensing means 200 illustrated in Figure 3, but wherein the inductances 2011, 2012, 2013 have been simplified and are schematically represented only with dotted lines, as are the connections 205, in order to bring forward the underlying connections 206 that are not clearly visible in Figure 3. In contrast to Figure 4, Figure 3 illustrates the inductances 2011, 2012, 2013 and the connections 205, but the underlying connections 206 are only shown in dotted lines to make clear that they are below the plane of Figure 3. The orientation of Figures 3 and 4 is otherwise essentially the same.

As illustrated in Figures 3 and 4, the parallel connections 205, 206 between the satellite inductances 2011, 2012, 2013 can be achieved using connecting elements 2051, 2052 and 2061, 2062, as well as connecting elements 2071, 2072, 2073. In Figure 3, the connecting elements 2051, 2052 are visible and are arranged towards the center 210, in particular around central area 220, and on the same plane as the inductances 2011, 2012, 2013, connecting respectively the outer pole of one of the inductances in the plurality, for instance here the outer pole 2042 of inductance 2012, to the respective outer pole of its neighboring inductances, here the poles 2041, 2043 of the two inductances 2011, 2013. As illustrated in Figure 3, following a preferred variant, the connection between the outer poles 2041 and 2043 can be left open, thereby forming an open loop. In less preferred embodiments, it would be still possible to close the connection between the outer poles 2041, 2043, but the open loop configuration is more advantageous than a closed loop, as will be explained hereafter. As further illustrated in Figure 3, the connecting elements 2051, 2052 can be arc-shaped. Furthermore, the connecting elements 2051, 2052 can also be essentially in the same plane as the inductances 2011, 2012, 2013 and can therefore also be provided in or on the same carrier substrate.

As shown in Figure 4, the inner poles 2031, 2032, 2033 of the inductances 2011, 2012, 2013 can in turn be connected via connecting elements 2071, 2072, 2073 that extend from the connecting elements 2061, 2062 towards the extremities 2081, 2082, 2083 contacting the inner poles 2031, 2032, 2033. It is preferred, but not necessary, that the connecting elements 2061, 2062 are arc-shaped like connecting elements 2051, 2052. It is also preferred, but not necessary, that the connecting elements 2061, 2062 are also arranged towards the center 210, in particular around central area 220. Together with the linear-shaped connecting elements 2071, 2072, the arc-shaped connecting elements 2061, 2062 can thus form a bridge-like structure 206 that connects the inner poles 2031, 2032, 2033 to one another.

In the embodiment illustrated in Figures 3 and 4, the arc-shaped 2061, 2062 and the linear-shaped 2071, 2072, 2073 connecting elements can be provided below the inductances 2011, 2012, 2013, as made clear by the dotted lines in Figure 3. For instance, they can be provided in or on a different carrier substrate as the inductances 2011, 2012, 2013 and connecting elements 2051, 2052, or they could be provided on the other side of the same carrier substrate. As further illustrated, the inner pole of one of the inductances, here the inner pole 2031 of inductance 2011, is connected to the respective inner poles of its neighboring inductances, here the poles 2032, 2033 of the two neighboring inductances 2012, 2013, respectively by means of the linear-shaped bridge element 2071 and the arc-shaped bridge element 2061 and the linear-shaped connecting element 2072 on one side, and the arc-shaped connecting element 2062 and the linear-shaped connecting element 2073 on the other side. Like for the connections 2051, 2052, following the same preferred variant, the connection 206 can be left open between the inner poles 2032, 2033, so that there is no arc-shaped connecting element closing the loop between connecting elements 2061, 2062, thereby forming an open loop in the parallel connection 205, 206. As explained above, in less preferred embodiments, it would be still possible to close the connection between the inner poles 2032, 2033, but the open loop configuration is more advantageous than a closed loop, as will be explained hereafter.

By leaving the parallel connections 205, 206 open as described above and as illustrated in Figures 3 and 4, the invention allows advantageously to prevent the undesired formation of Foucault currents, also known as eddies, in the passive sensing means 200, which would introduce unwanted perturbations in the monitoring, such as a weakening of the amplitude of the resonance signal. This has been found advantageous with respect to fully closed parallel connections and has been also found to improve the sensitivity of the passive sensing means 200 with respect to passive sensors for intraocular pressure monitoring known in the art.

Like in the passive sensing means 100 according to the first aspect of the invention, one or more capacitors could be added to the passive sensing means 200 to shift the resonance frequency and/or improve the signal-to-noise in the measurements. They are, however, not necessary to carry out the present invention, which is another advantage compared to existing passive sensors.

The passive sensing means 200 according to the first embodiment of the second aspect of the invention illustrated in Figures 3 and 4 is advantageous over passive sensors known in the art, as its design allows, once manufactured, curved, and finally incorporated in a contact lens, that the lens can be placed flat against the surface of a patient's eyeball while leaving sufficient flexibility to the contact lens to respond to deformations of the surface of the eye. Thus, variations of the intraocular pressure can be detected more efficiently than in sensing means known in the art. Further advantages of the inventive passive sensing means 200 will become obvious in combination with the embodiments illustrated in Figures 5 and 6 and described hereafter.

Figure 5 illustrates a passive sensing means 300 for a contact lens of a physiological parameter monitoring system, in a second embodiment of the second aspect of the invention. This second embodiment is similar in almost all aspects to the first embodiment illustrated in Figures 3 and 4 and comprises essentially the same optional features. It is therefore referred back to the description above for more details. The description of the second embodiment will therefore be focused more on the differences with respect to the first embodiment.

While the passive sensing means 200 of the first embodiment comprises three satellite inductances 2011, 2012, 2013, the passive sensing means 300 of the second embodiment illustrated in Figure 5 comprises an inductive element 301 with four satellite inductances 3011, 3012, 3013, 3014, spiraling also from their respective inner pole 3021, 3022, 3023, 3024 near their respective center 3031, 3032, 3033, 3034, towards their outer pole 3041, 3042, 3043, 3044. Like in the first embodiment, the inductances 3011, 3012, 3013, 3014 can be arranged at regular intervals from one another, preferably at 360 / 4 degrees = 90° from each other, as shown in Figure 5. The spiraling direction is not limitative and can be different from one embodiment to another, and even within a passive sensing means, it can be different from one inductance to another. For instance, with the spiraling direction being given from the inner pole towards the outer pole, the three satellite inductances 2011, 2012, 2013 of the first embodiment can spiral outwards counterclockwise, as illustrated in Figures 3 and 4. However, Figure 5 illustrates that, in the second embodiment, while two satellite inductances, here the inductances 3011, 3012, can spiral outwards counterclockwise, the other two satellite inductances, here the inductances 3013, 3014, can spiral outwards clockwise. It is, however, more advantageous and therefore preferred that all the inductances of a given passive sensing means spiral in the same direction and hence generate outgoing currents exiting through a same first node and ingoing currents entering through a same second node, different from the first node of the inductive element 301, as this increases and even maximizes the response or sensitivity of the passive sensing means to the external magnetic field.

Also like in the first embodiment, a parallel connection 305, 306 can also be preferred for the passive sensing means 300 of the second embodiment, with arc-shaped connecting elements 3051, 3052, 3053 having the same function as the connecting elements 2051, 2052 in the first embodiment and being therefore preferably provided on the same plane as the four inductances 3011, 3012, 3013, 3014, preferably on the same side of a carrier substrate 330 on which the inductances 3011, 3012, 3013, 3014 can also be provided.

Furthermore, either on a different carrier substrate or on the other side of carrier substrate 330, the preferably parallel connection 305, 306 can also comprise an underlying bridge-like connection 306 between the respective inner poles 3031, 3032, 3033, 3034, comprising three arc-shaped connecting elements and four linear-shaped connecting elements by analogy to the connection 206 with its two arc-shaped connecting elements 2061, 2062 and its three linear-shaped connecting elements 2071, 2072, 2073 described in the first embodiment with reference in particular to Figure 4.

In particular, since the connection 305, 306 can be a parallel connection, like in the first embodiment, the connection between two successive satellite inductances can preferably be left open. Figure 5 illustrates for instance that, in the second embodiment, the upper connection 305 can be left open between outer poles 3041 and 3044. By analogy to the first embodiment, the connection 306 can then be left open preferably between inner poles 3033 and 3034. This configuration has been found advantageous, as it avoids the formation of Foucault currents in the passive sensing means 300.

In the second embodiment, the passive sensing means 300 illustrated in Figure 5 is used in a method for manufacturing a contact lens for a physiological parameter monitoring system. Thus, following the inventive method, in the second embodiment, once the passive sensing means 300 is provided, a contact lens is subsequently over-molded over said passive sensing means 300. As a result, the passive sensing means 300 is incorporated between the internal and the external optical surfaces of the contact lens, with its center 310 aligned with the optical axis of the lens and in particular between the internal and the external surface vertices. As the passive sensing means 300 can comprise the free central area 320, the vision of a subject wearing the contact lens will remain essentially unimpaired while the variations of the intraocular pressure will be monitored with the advantages described above.

Following a preferred variant of the inventive method, in the second embodiment of the second aspect of the invention, preferably before over-molding the contact lens, the passive sensing means 300 can undergo a deforming step. In particular, the passive sensing means 300 can be plastically deformed. As a result of the deforming step, the passive sensing means 300 can obtain a curved shape corresponding to a predetermined curvature of the contact lens after over-molding. It is preferable to carry out the deforming step prior to the over-molding step in particular in order to prevent the formation of ripples in the lens.

In systems known in the art, it has been observed that sensors create stiff areas that cause ripples and/or deformations preventing the lens from being correctly placed flat on the surface of a person's eyeball. In the second embodiment, however, the invention allows taking advantage of the slight rigidity of the four satellite inductances 3011, 3012, 3013, 3014. Indeed, in the deforming step, the four inductances 3011, 3012, 3013, 3014 can be partially folded essentially like flaps or ears that can maintain the subsequently over-molded lens essentially flat against the surface of the eye, in an analogous way to the concave arc-shaped segments 1011, 1012, 1013 of the passive sensing means 100 in the embodiment of the first aspect of the invention illustrated in Figures 1 and 2. A variant with evenly distributed inductances, like the four inductances 3011, 3012, 3013, 3014 in the second embodiment, or like the three inductances 2011, 2012, 2013 in the first embodiment of the second aspect of the invention, is advantageous over variants with irregular arrangements, as the regular distribution will, in particular in combination with inductances having all substantially the same shape and size, improve even more the contact between the internal optical surface of the lens and the surface of the eyeball.

In the second embodiment according to the second aspect of the invention, following yet another advantageous variant of the inventive method, the carrier substrate 330 in or on which the passive sensing means 300 is provided can be removed in preferred areas between the inductances 3011, 3012, 3013, 3014 in order to improve even more the subsequent deforming step, and, consequently, also the over-molding step. In particular, the carrier substrate 330 can be removed following a predetermined pattern 331, preferably leaving cut-out areas 3311, 3312, 3313, 3314 between the inductances 3011, 3012, 3013, 3014. In a variant, the carrier substrate 330 could also be removed in the central area 320.

As illustrated in Figure 5, in this second embodiment, the cut-out areas 3131, 3312, 3313, 3314 can follow radial segments 3321, 3322, 3323, 3324. The radial segments 3321, 3322, 3323, 3324 around each respective inductance 3011, 3012, 3013, 3014 can be joined by a respective circumferential segment 3331, 3332, 3333, 3334. Thus, after the removing step, the carrier substrate 330 and the four satellite inductances 3011, 3012, 3013, 3014 of the passive sensing means 300 can form essentially a petal-like structure 331 around the central area 320, as illustrated in Figure 5.

In other advantageous variants, instead of following straight radial segments 3321, 3322, 3323, 3324, the carrier substrate 330 could be removed between the inductances 3011, 3012, 3013, 3014 following curved segments. For instance, like in hot-air balloons or in methods for wrapping spheres with sheets, the unnecessary carrier substrate 330 in the areas 3311, 3312, 3313, 3314 between the satellite inductances 3011, 3012, 3013, 3014 could be removed following a curved petal-shaped pattern, preferably following projections onto the plane of the small circle of great circular arcs of the lens.

Thus, in the second embodiment of the second aspect, by removing the carrier substrate 330 in the areas 3311, 3312, 3313, 3314 between the inductances 3011, 3012, 3013, 3014, the formation of ripples is prevented in the carrier substrate 330 during the deforming step, thereby preventing even better the formation of ripples in the over-molded lens. As a consequence, the contact lens will be more flexible, will not have ripples or stiff areas than prevent a correct placement on the eye, and will be able to stay in position flat against the eye better than in systems known in the art.

Optionally, it is also possible to remove the carrier substrate even more in order to prevent even better the formation of ripples in the curved substrate and in the over-molded contact lens. Thus, as also illustrated in Figure 5, in the second embodiment, at least one indentation 3351, 3352, 3352, 3353, 3354 can be made in each of the radial segments 3321, 3322, 3323, 3324 of the cut-out areas 3311, 3312, 3313, 3314. Similarly, as further illustrated in Figure 5, at least one indentation 3361, 3362, 3363, 3364 can also be made in each of the circumferential segments 3331, 3332, 3333, 3334. Alternatively, more than one indentation can be made in each radial or circumferential segment to facilitate even more the subsequent deforming step and prevent even better the formation of ripples in the substrate 330 and/or in the contact lens. The indentations could also be cut out as a part of the deforming step, or after the deforming step, but prior to the over-molding step.

Figure 6 illustrates a third embodiment of the second aspect of the invention, wherein a carrier substrate 430 has been removed following a different pattern 431 around a passive sensing means 400. The passive sensing means 400 of this third embodiment is similar to the passive sensing means 300 of the second embodiment and comprises essentially the same optional and advantageous features as the first and second embodiments of the second aspect of the invention. It is therefore referred back to the description above for more details. The description of the third embodiment will therefore be focused more on the differences with respect to the first and second embodiments of this aspect of the invention.

As illustrated in Figure 6, the passive sensing means 400 of the third embodiment is very similar to that of the second embodiment and comprises also an inductive element 401 with four satellite inductances 4011, 4012, 4013, 4014 spiraling from an inner pole 4031, 4032, 4033, 4034 close to, or on, their center 4021, 4022, 4023, 4024 towards an outer pole 4041, 4042, 4043, 4044. In the third embodiment, the four satellite inductances 4011, 4012, 4013, 4014 can be counterclockwise spirals like in the first embodiment. Like in the second embodiment, the four satellite inductances 4011, 4012, 4013, 4014 of the inductive element 401 of the third embodiment can be arranged in a non-overlapping manner at regular intervals, preferably at 360 / 4 degrees = 90° from each other, as illustrated in Figure 6.

Also like in the first and second embodiments, Figure 6 illustrates that a parallel connection 405, 406 can be preferred for the inductive element 401 of the passive sensing means 400 of the third embodiment and can be arranged towards the central area 420 around a center point 410 of the passive sensing means 400, with connecting elements 4051, 4052, 4053 arranged like the connecting elements 3051, 3052, 3053 of the second embodiment and connecting the outer poles 4041, 4042, 4043, 4044. Furthermore, in the third embodiment, the inner poles 3031, 3032, 3033, 3034 can also be connected via an underlying connection 406 like the connection 306 of the third embodiment and analog to connection 206 of the first embodiment. This parallel connection 405, 406 can also preferably be left open, with the same advantage as in the first and second embodiments that the formation of Foucault currents in the passive sensing means 400 is prevented.

In the third embodiment of the second aspect, the passive sensing means 400 illustrated in Figure 6 is used in a method for manufacturing a contact lens for a physiological parameter monitoring system, with essentially analogous steps as in the second embodiment. However, instead of removing the carrier substrate 430 following the predetermined pattern 331 of the second embodiment, in the third embodiment, the carrier substrate 430 can be removed in areas 4311, 4312, 4313, 4314 between the inductances 4011, 4012, 4013, 4014 following the contour 431 of the inductances 4011, 4012, 4013, 4014. In fact, the carrier substrate 430 can be removed following the general contour 431 of the passive sensing means 400, including also, when applicable, the contour of the central area 420 with the connections 405, 406. A petal-shape structure 431 can thereby also be obtained, but rounder and therefore smoother and with even less carrier substrate 430 than in the second embodiment of the second aspect of the invention. Thus, in this third embodiment, the deforming step and consequently also the over-molding step are facilitated even more with respect to the prior art, and the placement of the subsequently over-molded contact lens flat against the surface of the eye is even better.

As explained above, the first, second and third embodiments of the second aspect of the invention can be combined. For instance, it is possible to provide the passive sensing means 200 of the first embodiment in or on a carrier substrate and manufacture a contact lens following preferred variants of the inventive method described in the second and third embodiments.

The various embodiments of the inventive passive sensing means and their variants according to the two aspects of the invention have the advantage over sensors known in the art that the over-molding of the lens will not result in stiffened areas or in ripples that will cause problems for placing and maintaining the lens against a person's eye. On the contrary, passive sensing means according to the two aspects of the invention will provide for a better incorporation in a contact lens, and subsequently also for a better placement of the lens against the eye. Thus, the present invention provides an improvement in the field of passive sensing devices for monitoring variations of physiological parameters in a person's eye, for instance for patients suffering from glaucoma. In particular, the invention provides a contact lens that can be placed flat against a subject's eyeball, improving thereby also the sensitivity of the physiological parameter monitoring system.

## Claims

1. Passive sensing means (100) for a contact lens, in particular for detecting variations of a physiological parameter, comprising an inductance (101) having a plurality of concave arc-shaped segments (1011, 1012, 1013), preferably three, with respect to a center point (110) of the sensing means (100),
**characterized in that**
for at least one, preferably all, of the plurality of concave arc-shaped segments (1011, 1012, 1013) of the inductance (101), the radius of curvature of said at least one segment (1011, 1012, 1013) at a point of said segment (1011, 1012, 1013) is greater than the distance between said point and the center point (110) of the passive sensing means (101).

2. Passive sensing means (100) according to claim 1, wherein the inductance (101) further comprises convex arc-shaped segments (1021, 1022, 1023) arranged between the concave arc-shaped segments (1011, 1012, 1013).

3. Passive sensing means (100) according to claim 2, wherein the inductance (101) further comprises straight segments (1031, 1032, 1033) joining the convex arc-shaped segments (1021, 1022, 1023) to the concave arc-shaped segments (1011, 1012, 1013).

4. Passive sensing means (100) according to claim 3, wherein the junctions (1041, 1042, 1043, 1044, 1045, 1046) between the straight segments (1031, 1032, 1033) and the concave arc-shaped segments (1011, 1012, 1013) are rounded.

5. Passive sensing means (100) according to any of the preceding claims, wherein the inductance (101) is a spiral inductance, in particular comprising 5 to 20 spires, preferably 8 to 15 spires, more preferably 10 to 13 spires.

6. Passive sensing means (100) according to claim 5, wherein the width of the spires and/or the distance between spires is in a range from about 30 µm to about 100 µm, preferably about 40 µm to about 80 µm.

7. Passive sensing means (100) according to any of the preceding claims, wherein the width of the inductance (101) is about 2 mm or less, preferably about 1.5 mm or less.

8. Passive sensing means (100) according to any of the preceding claims, wherein the inductance (101) is provided in or on a carrier substrate (130), and wherein the carrier substrate (130) is removed following a predetermined pattern (131, 132), preferably following an external contour (131) and/or an internal contour (132) of the passive sensing means (100).

9. Passive sensing means (100) according to claim 8 in combination with any of claims 3 or 4, wherein more substrate (130) is left in areas (1331, 1332, 1333, 1334, 1335, 1336) of an internal contour (132) corresponding to the junction between the convex arc-shaped segments (1021, 1022, 1023) and the concave arc-shaped segments (1011, 1012, 1013).

10. Passive sensing means (200) for a contact lens, in particular for detecting variations of a physiological parameter, comprising an inductive element (201) having a plurality of inductances (2011, 2012, 2013),
**characterized in that**
the inductances (2011, 2012, 2013) are spaced apart from one another, preferably in a non-overlapping manner, in particular in a projection onto the small circle of the contact lens.

11. Passive sensing means (200) according to claim 10, wherein the inductances (2011, 2012, 2013) are off-centered with respect to a center point (210) of the sensing means (200), and in particular, with respect to the center of the small circle in the projection.

12. Passive sensing means (200) according to any of claims 10 or 11, wherein the inductances (2011, 2012, 2013) are arranged on the same side of a carrier substrate.

13. Passive sensing means (200) according to any of claims 10 to 12, wherein the inductances (2011, 2012, 2013) are connected to each other with the connections (2021, 2022, 2023) being arranged towards the center (210) of the sensing means.

14. Passive sensing means (200) according to claim 13, wherein the inductances (2011, 2012, 2013) are connected in parallel (205, 206) such that, when the inductances (2011, 2012, 2013) are placed in an external magnetic field, the outgoing currents generated by the inductances (2011, 2012, 2013) exit through a same first node, while the ingoing currents enter through a same second node, different from the first node.

15. Passive sensing means (200) according to claim 14, wherein at least one of the connections (205, 206) is left opened.

16. Passive sensing means (200) according to any of claims 10 to 15, wherein the inductances (2011, 2012, 2013) are spiral inductances, in particular circular spiral inductances, more in particular having substantially the same size.

17. Passive sensing means (300, 400) according to any of claims 10 to 16 in combination with claim 12, wherein the carrier substrate (330) is removed at least in areas (3311, 3312, 3313, 3314) between the inductances (3011, 3012, 3013, 3014) following a predetermined pattern (331, 431), preferably following the contour (431) of the passive sensing means (400).

18. Contact lens for a physiological parameter measuring system comprising a passive sensing means (100, 200, 300, 400) according to any of claims 1 to 17.

19. Method for manufacturing a contact lens according to claim 18 comprising the steps of:
providing a passive sensing means (100, 200, 300, 400) according to any of claims 1 to 17; and
over-molding the contact lens over the passive sensing means (100, 200, 300, 400), such that the passive sensing means (100, 200, 300, 400) is incorporated in said contact lens between an internal optical surface and an external optical surface of the contact lens,
wherein the internal optical surface is the surface for contacting a person's eyeball, and the external optical surface is the outer surface opposite the internal surface,
further comprising, preferably prior to the step of over-molding the contact lens, a step of deforming, in particular plastically, the passive sensing means (100, 200, 300, 400) to obtain a curved shape corresponding to the shape of the contact lens.

20. Method according to claim 19, in combination with a passive sensing means (100) according to any of claims 8 or 9, or in combination with a passive sensing means (300, 400) according to claim 17, further comprising, prior to the step of over-molding the contact lens or to the step of deforming the passive sensing means, a step of removing the carrier substrate (130, 330, 430) following the predetermined pattern (131, 132, 331, 431).
